# EUROPÄISCHE PATENTSCHRIFT

(11) **EP 2 781 211 B1**
(45) Veröffentlichungstag und Bekanntmachung des Hinweises auf die Patenterteilung: **03.05.2017**
(21) Anmeldenummer: 14151131.1
(22) Anmeldetag: 14.01.2014
(51) Int. Cl.: A61K 8/37, A61K 8/58, A61Q 17/04, A61K 8/06

(54) **Stabile kosmetische Zubereitung**
Stable cosmetic preparation
Préparation cosmétique stable

(30) Priorität: 01.03.2013 DE 102013203559
(43) Veröffentlichungstag der Anmeldung: 24.09.2014
(73) Patentinhaber: Beiersdorf AG, 20253 Hamburg (DE)
(72) Erfinder: Johns, Nicole, 22958 Kuddewörde (DE); Blohm, Alexandra, 22607 Hamburg (DE)

(56) Entgegenhaltungen:
- EP-A1- 1 541 152
- EP-A1- 1 579 848
- EP-A2- 2 382 961
- DE-A1-102011 002 863
- DATABASE GNPD [Online] MINTEL; 30. April 2007 (2007-04-30), "Anti-Ageing Skincare Pack", XP002728333, Database accession no. 682790
- DATABASE GNPD [Online] MINTEL; 31. Januar 2009 (2009-01-31), "3 Step Firming & Lifting Kit", XP002728334, Database accession no. 1027895

## Beschreibung

Die vorliegende Erfindung betrifft eine kosmetische Zubereitung enthaltend Ethylhexylcocoat und Silica Dimethyl Silylate.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.EP 2382961 offenbart kosmetische Zusammensetzungen zur optischen Kaschierung von Falten. Diese Zusammensetzungen liegen in Form einer Öl-in-Wasser Emulsion vor. Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Um kosmetische Sonnenschutzmittel mit hohem Lichtschutzfaktor herzustellen, müssen nach dem Stand der Technik relativ hohe Mengen an -in ihrer Herstellung relativ aufwendigen und damit teuren- UV-Filtern eingearbeitet werden. Diese in der Regel lipophilen (teilweise bei Raumtemperatur flüssigen) UV-Filter führen in hoher Einsatzkonzentration dazu, das die Zubereitungen relativ schwer in die Haut einziehen und damit einen relativ "schweren", schmierig-klebrigen sensorischen Eindruck nach dem Auftragen auf der Haut hinterlassen. Die Verbraucher hingegen wünschen "leichte" Formeln, bei denen man nach einer kurzen Einwirkungszeit nicht mehr spürt, dass sie auf die Haut aufgetragen wurden.

Um kosmetische Zubereitungen im allgemeinen und speziell Sonnenschutzmittel sensorisch attraktiver zu gestalten, sollte daher die Einsatzkonzentration der UV-Filter möglichst gering sein und der Zubereitung Puderstoff-artige Sensorik-Additive zugesetzt werden.

Ein Beispiel für einen solches Additiv stellen die auch als Aerosile bekannten Silica Dimethyl Silylate dar, die darüber hinaus den Vorteil haben, den Lichtschutzfaktor der Zubereitung weiter zu verstärken.

Nachteilig am Stande der Technik, d.h. an Zubereitungen , welche Silica Dimethyl Silylate enthalten, ist jedoch der Umstand, dass dieser Inhaltsstoff in Emulsionen nicht besonders stabil ist und sich bei längerer Lagerung, unter Erschütterungen (Schütteltest) und bei Temperaturschwankungen leicht aus der Ölphase der Emulsion, in welche er eingearbeitet wird, wieder abscheidet und ausfällt. Zwar können Emulgierverfahren, die mit hohem Energieeintrag arbeiten, diesen Effekt etwas unterdrücken, doch stellt die stabile Einarbeitung von Silica Dimethyl Silylate immernoch ein großes Problem bei der Verwendung dieses Inhaltsstoffes dar.

Es war daher die Aufgabe der vorliegenden Erfindung, Mittel und Wege zu finden, Silica Dimethyl Silylate möglichst energiesparend stabil in Emulsionen, insbesondere Sonnenschutzmittel einzuarbeiten.

Überraschend gelöst wird die Aufgabe durch eine kosmetische Zubereitung enthaltend
a) Ethylhexylcocoat und
b) Silica Dimethyl Silylate, dadurch gekennzeichnet, dass die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethyl-propyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine.

Wie sich aus den Vergleichsversuchen nicht vorhersehbar ergab, ist Ethylhexylcocoat, im Gegensatz zu anderen Ölkomponenten wie beispielsweise Triisostearrin oder C13-16 Isoparaffin, in der Lage, Silica Dimethyl Silylate in der Ölphase und der Gesamtemulsion deutlich zu stabilisieren.

Erfindungsgemäß ist daher auch die Verwendung von Ethylhexylcocoat zur Stabilisierung von kosmetischen Zubereitungen enthaltend Silica Dimethyl Silylate.

Zwar kennt der Stand der Technik die GNPD Mintel Datenbank Einträge "Anti-Aging Skincare, Pack", XP002728333 vom 30. April 2007 (Database accession no. 682790) und "3 Step Firming & Lifting Kit", XP002728334 vom 31.Januar 2009 (Database accession no. 1027895), sowie die EP2382961, EP1579848, EP 1541152 und DE 102011002863, doch konnten diese Schriften nicht den Weg zur vorliegenden Erfindung weisen.

Es ist erfindungsgemäß vorteilhaft, wenn die erfindungsgemäße Zubereitung Ethylhexylcocoat in einer Konzentration von 1,5 bis 6,0 Gewichts-%, bezogen auf das Gesamtgewicht der Zubereitung enthält.

Es ist erfindungsgemäß bevorzugt, wenn die erfindungsgemäße Zubereitung Ethylhexylcocoat in einer Konzentration 0,5 von bis 10 Gewichts-% , bezogen auf das Gesamtgewicht der Zubereitung enthält.

Es ist erfindungsgemäß vorteilhaft, wenn die erfindungsgemäße Zubereitung Silica Dimethyl Silylate in einer Konzentration von 0,1 bis 0,7 Gewichts-%, bezogen auf das Gesamtgewicht der Zubereitung enthält.

Es ist erfindungsgemäß bevorzugt, wenn die erfindungsgemäße Zubereitung Silica Dimethyl Silylate in einer Konzentration von 0,05 bis 2,0 Gewichts-%, bezogen auf das Gesamtgewicht der Zubereitung enthält.

Als erfindungsgemäß besonders bevorzugtes Silica Dimethyl Silylate kann beispielsweise der Rohstoff Aerosil 972 der Firma Evonik eingesetzt werden.

Es ist erfindungsgemäß vorteilhaft, wenn die erfindungsgemäße Zubereitung eine Emulsion darstellt. Es ist erfindungsgemäß bevorzugt, wenn es sich bei der erfindungsgemäße Zubereitung um eine O/W-Emulsion handelt.

Im Falle der O/W-Emulsion ist es erfindungsgemäß bevorzugt, wenn die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, Natriumcetearylsulfat, Kaliumcetylphosphat enthält.

Erfindungsgemäß besonders bevorzugt ist es, wenn die Zubereitung Natriumstearylglutamat und/oder Polyglyceryl-3 Methylglucose Distearat enthält.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Dabei betreffen die Konzentrationsangaben die Einzelkonzentrationen der O/W-Emutgatoren.

Erfindungsgemäß vorteilhafte Ausführungsformen der vorliegenden Erfindung sind dadurch gekennzeichnet, dass die Zubereitung ein oder mehrere UV-Filter enthält.

Erfindungsgemäß bevorzugt handelt es sich bei der erfindungsgemäßen Zubereitung um ein Sonnenschutzmittel.

Es ist erfindungsgemäß, wenn die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethyl-propyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine.

Diese können in einer Gesamtmenge von 0,1-40 Gewichts-%, bezogen auf das Gesamtgewicht der Zubereitung, in dieser enthalten sein.

Erfindungsgemäß bevorzugt ist die erfindungsgemäße Zubereitung frei von 3-(4-Methylbenzyliden)campher.

Es ist erfindungsgemäß bevorzugt, wenn die erfindungsgemäße Zubereitung Octyldodecanol enthält.

Es ist erfindungsgemäß vorteilhaft, wenn die erfindungsgemäße Zubereitung Octyldodecanol in einer Konzentration von 1,0 bis 7,0 Gewichts-%, bezogen auf das Gesamtgewicht der Zubereitung enthält.

Es ist erfindungsgemäß bevorzugt, wenn die erfindungsgemäße Zubereitung Octyldodecanol in einer Konzentration von 0,5 bis 10,0 Gewichts-%, bezogen auf das Gesamtgewicht der Zubereitung enthält.

Erfindungsgemäß vorteihafte Ausführungsformen der vorliegenden Erfindung sind dadurch gekennzeichnet, dass die Zubereitung einen oder mehrere Wirkstoffe gewählt aus der Gruppe der Verbindungen Glycyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, enthält.

Ferner ist es erfindungsgemäß von Vorteil, wenn die Zubereitung Methylparaben, Ethylparaben,Propylparaben, 2-Methylisothiazol-3(2*H*)-on und/oder Phenoxyethanol enthält.

Auch kommt man zu erfindungsgemäß vorteilhaften Ausführungsformen, wenn die erfindungsgemäße Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält.

Erfindungsgemäß vorteilhafte Ausführungsformen der vorliegenden Erfindung sind dadurch gekennzeichnet, dass die Zubereitung Acrylat/C10-30 Alkylacrylat Crosspolymer (INCI Acrylates/C10-30 Alkylacrylate Crosspolymer) und/oder Xanthangummi (INCI Xanthan Gum) enthält.

Es ist erfindungsgemäß vorteilhaft, wenn die erfindungsgemäße Zubereitung Acrylat/C10-30 Alkylacrylat Crosspolymer in einer Konzentration von 0,05 bis 0,4 Gewichts-%, bezogen auf das Gesamtgewicht der Zubereitung enthält.

Es ist erfindungsgemäß bevorzugt, wenn die erfindungsgemäße Zubereitung Acrylat/C10-30 Alkylacrylat Crosspolymer in einer Konzentration von 0,01 bis 1,0 Gewichts-%, bezogen auf das Gesamtgewicht der Zubereitung enthält.

Es ist erfindungsgemäß vorteilhaft, wenn die erfindungsgemäße Zubereitung Xanthangummi in einer Konzentration von 0,2 bis 0,6 Gewichts-%, bezogen auf das Gesamtgewicht der Zubereitung enthält.

Es ist erfindungsgemäß bevorzugt, wenn die erfindungsgemäße Zubereitung Xanthangummi in einer Konzentration von 0,1 bis 1,0 Gewichts-%, bezogen auf das Gesamtgewicht der Zubereitung enthält.

Erfindungsgemäß vorteilhaft enthält die erfindungsgemäße Zubereitung ein oder mehrere Parfümstoffe. Dabei sind diese Zubereitungen erfindungsgemäß bevorzugt dadurch gekennzeichnet, dass die Zubereitung frei ist von Lyral.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner (z.B. Dihydroxyaceton), Repellentien sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß vorteilhaft enthält die erfindungsgemäße Zusammensetzung ein oder mehrere Feuchthaltemittel (Moisturizer). Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Hydroxyethylharnstoff, Glycine Soja, Ethylhexyloxyglycerin, Pyrrolidoncarbonsäure, Glycerylglucose Quaternäre Verbindungen, Alkandiole und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 15 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Polysilsesquioxane, Siloxanelastomere, Aluminium- bzw. Natrium-Stärke Octenylsuccinat und dergleichen), Pigmente, die weder hauptsächlich UV-Filter- noch färbende Wirkung haben (wie z. B. Bornitrid etc.), Polyethylen, Nylon, Aerosile^{®} (CAS-Nr. 7631-86-9) und /oder Talkum.

Erfindungsgemäß bevorzugt ist es, wenn die Zubereitung Füllstoffe, insbesondere Polyethylen, Nylon, natürliche oder modifizierte Stärken wie Tapiokastärke und/oder Silikate wie beispielsweise Talkum enthält.

### Vergleichsversuche

Es wurden die folgenden Emulsionen hergestellt und ein "Schütteltest" durchgeführt:

### Durchführung Schütteltest:

Diese Methode wurde evaluiert, um frühzeitig mögliche Instabilitäten von Rezepturen durch den Transport festzustellen.

Der Schütteltest ist eine Methode, die als Hinweis auf mögliche Ölabscheidungen dient. Ölabscheidungen können häufig bei kosmetischen Emulsionen auftreten.

### Durchführung/ Parameter:

Gerät: Edmund Bühler KS-15 Control
Frequenz: 350 U/min
Zeit: 24 h
Probengefäß: 50mL PE-Flasche, 30mL Füllhöhe
Kriterien: Ölabscheidung, Frequenz (U/min)
Lagerort: Raumtemperatur

**Rezepturen**

| INCI | Rezeptur 1 | Rezeptur 2 |
|---|---|---|
| Homosalate | 7 | 7 |
| Ethylhexyl Salicylate | 3,5 | 3,5 |
| Butyl Methoxydibenzoylmethane | 3,5 | 3,5 |
| Titanium Dioxide + Trimethoxycaprylylsilane | 1,5 | 1,5 |
| Octocrylene | 7 | 7 |
| Glycyrrhetinic Acid | 0,5 | 0,5 |
| Octyldodecanol | 4 | 4 |
| Caprylic/Capric Triglyceride | 1 | 1 |
| Glyceryl Stearate | 1 | 1 |
| Hydrogenated Coco-Glycerides | 1 | 1 |
| **Ethylhexyl Cocoate** | **4** | |
| **Triisostearin** | | **4** |
| **C13-16 Isoparaffin** | | |
| Sodium Stearoyl Glutamate | 0,3 | 0,3 |
| Silica Dimethyl Silylate | 0,3 | 0,3 |
| Parfum | 0,4 | 0,4 |
| Aqua + Sodium Hydroxide | 0,025 | 0,025 |
| Phenoxyethanol | 0,6 | 0,6 |
| Methylparaben | 0,3 | 0,3 |
| Acrylates/C10-30 Alkyl Acrylate Crosspolymer | 0,1 | 0,1 |
| Xanthan Gum | 0,5 | 0,5 |
| Aqua | 57,475 | 57,475 |
| Alcohol Denat. | 6 | 6 |

| **Stabilitätsdaten** | Rezeptur 1 | Rezeptur 2 |
|---|---|---|
| **Schütteltest 1d bei 350U/min.** | 100% | 90% (minimal Öl auf Oberfläche) |
| **B40GL n.14d Beurteilung bei 40°C Lagerung im Glas** | 90% (minimal Öl auf Oberfläche) | 70 (leichte Ölabscheidung) |

### Visuelle Beurteilung:

Mit Ethylhexyl Cocoate entstehen stabile Emulsionen. Unter Verwendung der anderen Lipiden kommt es zu Ölabscheidungen.

### Beispiele:

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Rezepturen**

| INCI | Rezeptur 1 |
|---|---|
| Homosalate | 7 |
| Ethylhexyl Salicylate | 3,5 |
| Butyl Methoxydibenzoylmethane | 3,5 |
| Titanium Dioxide + Trimethoxycaprylylsilane | 1,5 |
| Octocrylene | 7 |
| Glycyrrhetinic Acid | 0,5 |
| Octyldodecanol | 4 |
| Caprylic/Capric Triqlyceride | 2 |
| Glyceryl Stearate | 1 |
| Hydrogenated Coco-Glycerides | 1 |
| Ethylhexyl Cocoate | 4 |
| Sodium Stearoyl Glutamate | 0,3 |
| Silica Dimethyl Silylate | 0,3 |
| Parfum | 0,3 |
| Sodium Hydroxide | 0,025 |
| Phenoxyethanol | 0,6 |
| Methylparaben | |
| Acrylates/C10-30 Alkyl Acrylate Crosspolymer | 0,1 |
| Xanthan Gum | 0,5 |
| Alcohol denat | 4,0 |
| Aqua | Ad 100,0 |

**Rezepturen**

| INCI | Rezeptur 2 |
|---|---|
| Homosalate | 7 |
| Ethylhexyl Salicylate | 3,5 |
| Butyl Methoxydibenzoylmethane | 3,5 |
| Titanium Dioxide + Trimethoxycaprylylsilane | 1,5 |
| Ethylhexyltriazone | 4,0 |
| Bis-Ethylhexyloxyphenol Methoxyphenyl Triazine | 1,0 |
| Diethylhexyl Butamido Triazone | 1,0 |
| Diethylamino Hydroxybenzoyl Hexyl Benzoate | 5,0 |
| Octyldodecanol | 4 |
| Caprylic/Capric Triplyceride | 1 |
| Glyceryl Stearate | 1 |
| Hydrogenated Coco- Glycerides | 1 |
| Ethylhexyl Cocoate | 4 |
| Sodium Stearoyl Glutamate | 0,3 |
| Silica Dimethyl Silylate | 0,3 |
| Parfum | 0,4 |
| Sodium Hydroxide | 0,025 |
| Phenoxyethanol | 0,6 |
| | |
| Acrylates/C10-30 Alkyl Acrylate Crosspolymer | 0,1 |
| Xanthan Gum | 0,5 |
| Alcohol denat | 6,0 |
| Aqua | Ad 100,0 |

## Patentansprüche

1. Kosmetische Zubereitung enthaltend
a) Ethylhexylcocoat und
b) Silica Dimethyl Silylate, **dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine.

2. Kosmetische Zubereitung nach Anspruch 1 **dadurch gekennzeichnet, dass** es sich um eine O/W-Emulsion handelt.

3. Kosmetische Zubereitung, **dadurch gekennzeichnet, dass** die Zubereitung Natriumstearylglutamat und/oder Polyglyceryl-3 Methylglucose Distearat enthält.

4. Kosmetische Zubereitung nach einem der vorhergehenden Ansprüche, **dadurch gekennzeichnet, dass** die Zubereitung Octyldodecanol enthält.

5. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, enthält.

6. Kosmetische Zubereitung nach einem der vorhergehenden Ansprüche, **dadurch gekennzeichnet, dass** die Zubereitung Methylparaben, Ethylparaben,Propylparaben, 2-Methylisothiazol-3(2*H*-on und/oder Phenoxyethanol enthält.

7. Kosmetische Zubereitung nach einem der vorhergehenden Ansprüche, **dadurch gekennzeichnet, dass** die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält.

8. Kosmetische Zubereitung nach einem der vorhergehenden Ansprüche, **dadurch gekennzeichnet, dass** die Zubereitung Acrylat/C10-30 Alkylacrylat Crosspolymer und/oder Xanthangummi enthält.

9. Kosmetische Zubereitung nach einem der vorhergehenden Ansprüche, **dadurch gekennzeichnet, dass** die Zubereitung frei ist von Lyral.

## Claims

1. Cosmetic preparation comprising
a) ethylhexyl cocoate und
b) silica dimethyl silylate, **characterized in that** the preparation comprises one or more UV filters selected from the group of compounds comprising 2- phenylbenzimidazole-5-sulphonic acid and/or salts thereof; phenylene-1,4-bis(2-benzimidazyl)-3,3'-5,5'-tetrasulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4- (1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 4-(tert-butyl)-4'-methoxydibenzoylmethane; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydoxybenzoyl)benzoate; homomenthyl salicylate; 2-ethylhexyl 2-hydroxybenzoate; dimethicodiethylbenzalmalonate; 3-(4-(2,2-bisethoxycarbonylvinyl)phenoxy)propenyl)methoxysiloxane/dimethyfsiloxan e copolymer; dioctylbutylamidotriazone (INCI: Diethylhexyl Butamido Triazone); 2,4-bis[5-1 (dimethylpropyl)benzoxazol-2-yl(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine; 2,4,6-tribiphenyl-4-yl-1,3,5-triazire; merocyanine.

2. Cosmetic preparation according to Claim 1, **characterized in that** said preparation is an O/W emulsion.

3. Cosmetic preparation, **characterized in that** the preparation comprises sodium stearyl glutamate and/or polyglyceryl-3 methylglucose distearate.

4. Cosmetic preparation according to any of the preceding claims, **characterized in that** the preparation comprises octyldodecanol.

5. Cosmetic preparation according to any of the preceding claims, **characterized in that** the preparation comprises one or more active ingredients selected from the group of compounds glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, β-alanine and/or licochalcone A.

6. Cosmetic preparation according to any of the preceding claims, **characterized in that** the preparation comprises methylparaben, ethylparaben, propylparaben, 2-methylisothiazol-3(*2H*)-one and/or phenoxyethanol.

7. Cosmetic preparation according to any of the preceding claims, **characterized in that** the preparation comprises propylene glycol, butylene glycol, 2-methylpropane-1,3-diol, 1,2-pentanediol, 1,2-hexanediol, 1,2-octanediol and/or 1,2-decanediol.

8. Cosmetic preparation according to any of the preceding claims, **characterized in that** the preparation comprises acrylate/C10-30 alkyl acrylate crosspolymer and/or xanthan gum.

9. Cosmetic preparation according to any of the preceding claims, **characterized in that** the preparation is free of Lyral.

## Revendications

1. Préparation cosmétique contenant
a) du cocoate d'éthylhexyle et
b) du Silica Dimethyl Silylate, **caractérisée**
**en ce que** la préparation contient un ou plusieurs filtres UV, choisis dans le groupe formé par les composés acide 2-phénylbenzimidazole-5-sulfonique et/ou ses sels; sels de l'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidèneméthyl)benzènesulfonique, sels de l'acide 2-méthyl-5-(2-oxo-3-bornylidèneméthyl)sulfonique; 2,2'-méthylène-bis-(6-(2H-benzotriazol-2-yl)-4-(1,1,3,3-tétraméthylbutyl)-phénol); 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre; 3-benzylidènecamphre; salicylate d'éthylhexyle ; acide téréphtalidènedicamphresulfonique; 4-(tert-butyl)-4'-méthoxydibenzoylméthane; 2-cyano-3,3-diphénylacrylate de 2-éthylhexyle; ester 2-éthylhexylique de l'acide 4-(diméthylamino)-benzoïque; ester amylique de l'acide 4-(diméthylamino)benzoïque ; ester di-(2-éthylhexylique) de l'acide 4-méthoxybenzalmalonique; ester 2-éthylhexyüque de l'acide 4-mélhoxycinnamique; ester isoamylique de l'acide 4-méthoxycinnamique; 2-hydroxy-4-méthoxybenzophénone, 2-hydroxy-4-méthoxy-4'-méthylbenzophénone; 2,2'-dihydroxy-4-méthoxybenzophénone; ester hexylique de l'acide 2-(4'-diéthylamino-2'-hydroxybenzoyl)-benzoïque; salicylate d'homomenthyle; 2-hydroxybenzoate de 2-éthylhexyle; diméthicodiéthylbenzalmalonate; copolymère de 3-(4-(2,2-bis-éthoxycarbonylvinyl)-phénoxy)propényl)-méthoxysiloxane/diméthylsiloxane; dioctylbutylamidotriazone (INCI: Diethylhexyl-Butamidotriazone; 2,4-bis-[5-1 (diméthylpropyl)benzoxazol-2-yl-(4-phényl)-imino]-6-(2-éthylhexyl)-imino-1,3,5-triazine (présentant le n° CAS 288254-16-0) ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine (INCI: Ethylhexyl Triazone)); 2,4-bis-{[4-(2-éthylhexyloxy)-2-hydroxy]-phényl}-6-(4-méthoxyphényl)-1,3,5-triazine; 2,4,6-tribiphényl-4-yl-1,3,5-triazine; mérocyanines.

2. Préparation cosmétique selon la revendication 1, **caractérisée en ce qu'**il s'agit d'une émulsion H/E.

3. Préparation cosmétique, **caractérisée en ce que** la préparation contient du stéarylglutamate de sodium et/ou du Polyglyceryl-3 Methylglucose Distearate.

4. Préparation selon l'une quelconque des revendications précédentes, **caractérisée en ce que** la préparation contient de l'octyldodécanol.

5. Préparation selon l'une quelconque des revendications précédentes, **caractérisée en ce que** la préparation contient une ou plusieurs substances actives choisies dans le groupe des composés acide glycyrrhétinique, urée, arctiine, acide alpha-liponique, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, β-alanine et/ou licochalcone A.

6. Préparation selon l'une quelconque des revendications précédentes, **caractérisée en ce que** la préparation contient du méthylparabène, de l'éthylparabène, du propylparabène, de la 2-méthylisothiazol-3(2H)-one et/ou du phénoxyéthanol.

7. Préparation selon l'une quelconque des revendications précédentes, **caractérisée en ce que** la préparation contient du propylèneglycol, du butylèneglycol, du 2-méthylpropane-1,3-diol, du 1,2-pentanediol, du 1,2-hexanediol, du 1,2-octanediol et/ou du 1,2-décanediol.

8. Préparation selon l'une quelconque des revendications précédentes, **caractérisée en ce que** la préparation contient du Acrylat/C1 0-30 Alkylacrylat Crosspolymer et/ou de la gomme de xanthane.

9. Préparation selon l'une quelconque des revendications précédentes, **caractérisée en ce que** la composition est exempte de lyral.
